# EUROPEAN PATENT APPLICATION

(11) **EP 4 772 209 A1**
(43) Date of publication of application: **08.07.2026**
(21) Application number: 25796456.9
(22) Date of filing: 24.04.2025
(51) Int. Cl.: A61M 3/02

(54) **STOOL EXTRACTION DEVICE AND STOOL EXTRACTION SET PROVIDED WITH STOOL STORAGE BAG**

(30) Priority: 01.05.2024 JP 2024074303
(71) Applicant: Beppu, Yoshio, Tokyo 167-0022 (JP)
(72) Inventor: Beppu, Yoshio, Tokyo 167-0022 (JP)
(74) Representative: Isarpatent
(86) International application number: PCT/JP2025/015979
(87) International publication number: WO 2025/229925

(57) **Abstract**

The disimpaction device (10) includes an insertion part (14) that is inserted through the anus (11) so that its distal end (12) comes into contact with hard feces (13); a vibration generating unit (15) disposed outside the anus (11) for vibrating the distal end (12) of the insertion part (14); and a liquid supply unit (16) that supplies liquid from the distal end (12) to the hard feces (13). The insertion part (14) has flexibility that allows it to deform along the shape of the rectum, and the vibration of the distal end (12) mixes the hard feces (13) with the liquid to form slurry-like feces. When the storage bag is affixed to the buttocks, the elastic force of the elastic part for opening causes the opening of the bag body (46) to naturally expand, thereby allowing the discharged slurry-like feces to be collected therein.

## Description

### Technical Field

The present invention relates to a disimpaction device and a disimpaction set integrating a storage bag for storing feces extracted by the disimpaction device.

### Background Art

The current practice performed when a patient complains of constipation involves administering a glycerin enema or performing manual disimpaction. For example, the patient is placed in a lateral position, and the practitioner inserts an instrument or a finger into the patient's anus to remove the feces. However, when hard feces are firmly lodged in the anus, inserting a finger to scoop it out is not easy. When using an enema, the practitioner carefully inserts a resin tube attached to the distal end of the enema device approximately 10 cm into the anus and squeezes the container of the enema to inject glycerin into the rectum. During this process, glycerin often begins to ooze out of the anus. The practitioner quickly presses a rolled-up piece of paper or similar material against the anus and waits for the glycerin to act on the intestinal mucosa, causing the smooth muscles of the colon to contract and trigger a bowel movement. After about five to ten minutes, the patient may complain of abdominal pain and appear to experience intestinal spasms, which is perceived as pain. At that time, even if the anus is being pressed, more glycerin leaks out. Once the practitioner releases their hand, fecal masses coated with glycerin are expelled. If the feces stop coming out after a certain amount has been expelled, the practitioner inserts a finger into the anus to remove the remaining feces. During these processes, glycerin and feces frequently leak out and soil the bedding and the rubber gloves worn by the practitioner, and large amounts of used paper waste are generated. Meanwhile, deciding whether to administer a glycerin enema is not easy, because it is uncertain whether the patient will be able to defecate as a result of being administered the enema.

Various instruments have been proposed as alternatives to the above-mentioned methods for feces extraction. In the device disclosed in Patent Literature 1, a rigid disimpaction tube is inserted into the rectum to remove the feces. Apart from Patent Literature 1, other instruments are also known in which a rigid tubular member is inserted into the rectum to extract feces. In many of these proposals, fecal masses in solid form are accommodated in a tubular member for removal.

The disimpaction device described in Patent Literature 1 injects a lubricating liquid such as water from around the distal end of the disimpaction tube. Among conventional disimpaction devices that supply such a lubricating liquid, structures have also been proposed in which water is ejected in pulses from the distal end to break down the fecal mass. Patent Literature 3 proposes an insertion instrument for an excretion-processing device that uses ultrasound. In this device, an ultrasonic horn is placed inside a protective member that can expand outward in a horn shape, and this protective member is housed within an outer sheath in a contracted state. During use, the outer sheath is inserted into the rectum through the patient's anus, and the outer sheath is dissolved or decomposed to allow the protective member to expand. Then, by supplying water or other fluid to inside the protective member through a suction nipple and vibrating the ultrasonic horn while simultaneously performing suctioning through the suction nipple, the feces are fragmented and aspirated for removal.

### Citation List

### Patent Literature

Patent Literature 1: JP 2000-157628 A
Patent Literature 2: JP Utility Model Registration 3232394
Patent Literature 3: JP Utility Model Publication 1995-18738 U

### Summary of Invention

### Technical Problem

However, with instruments of the type that remove feces by inserting a rigid tube into the rectum, the tube must have a certain thickness, making it difficult to insert it into the patient, and also the manufacturing cost is high. Moreover, since the rectum following the anus is curving basically, a rigid tube is not suitable. Even if feces extraction could be performed with such an instrument, cleaning and reusing it after each use would not be easy. In the case of devices that supply water or other liquids to the feces, the procedure requires effort similar to that of an enema. Furthermore, devices that eject pulsed water involve risks because they spray high-pressure water into the colon, raising concerns about potential accidents such as colonic rupture.

Furthermore, with instruments that use ultrasonic waves, merely applying ultrasonic waves does not increase the fluidity of feces even if they can produce slight deformation on the surface of the fecal mass, and in practice feces extraction cannot be achieved. Constipation is frequently observed in elderly individuals, and in nursing homes, bowel management occupies a significant part of daily care tasks. In Japan, the population is aging at an accelerating pace, and the need for a disimpaction device that can be operated easily is increasing.

Accordingly, the present invention provides a disimpaction device that enables feces to be removed easily as well as a storage bag for collecting the extracted feces, and further provides a disimpaction set integrating these components as a unit.

### Solution to Problem

The inventor has been practicing as a clinician for nearly forty years, providing guidance to nurses and at times performing manual disimpaction himself. Based on this experience, and through careful study to solve the above-mentioned problems, the present invention has ultimately been conceived. The disimpaction device of the present invention includes: an insertion part that is inserted through the anus and has a distal end configured to contact hard feces; a vibration generating unit disposed outside the anus to vibrate the distal end of the insertion part; and a liquid supply unit for supplying liquid from the distal end of the insertion part to the hard feces. The insertion part is flexible so that, when inserted from the anus into the rectum, it can deform along the shape of the rectum, and it is configured to form slurry-like feces by mixing the hard feces with the liquid through vibration of the distal end. The vibration generating unit produces vibrations with an amplitude of approximately 1 to 5 mm, for example.

According to the disimpaction device of the present invention, the distal end of the insertion part is inserted through the anus and brought into contact with the hard feces, and liquid is supplied from the liquid supply unit. In this state, the vibration generating unit vibrates the distal end of the insertion part, thereby mixing the hard feces with the liquid. As a result, the hard feces present in the rectum and anus can be liquefied into slurry-like feces, which can then be easily discharged from the patient's anus. Moreover, because the insertion part that is inserted from the anus into the rectum has flexibility allowing it to deform along the shape of the rectum, it can be inserted into the patient's rectum easily and safely.

The liquid supply unit preferably includes a liquid supply source disposed outside the anus, and a flexible tube for guiding the liquid from the liquid supply source to the distal end of the insertion part. A vibration plate is arranged at the distal end of the insertion part, and the vibration plate is provided with a protruding piece that protrudes in the axial direction from the surface facing the hard feces so as to make direct contact with the hard feces. By the vibration of the vibration plate, the hard feces are mixed with the liquid, thereby forming slurry-like feces. The vibration plate is disposed at the distal end of the flexible tube and has a discharge port through which the liquid from the flexible tube is discharged. With this configuration, liquid can be supplied from outside of the anus to the distal end of the insertion part without fail, and by supplying liquid from the distal end while vibrating the hard feces, the hard fecal mass can be crushed and mixed with the liquid to form slurry-like feces.

The insertion part includes a flexible and vibration-capable transmission shaft connected to the vibration generating unit, and a vibration plate provided at the distal end of the shaft. Preferably, the transmission shaft is accommodated within a flexible tube, and the transmission shaft and the flexible tube are interchangeably connected to the vibration generating unit. With this configuration, the vibration generated by the vibration generating unit can be transmitted to the vibration plate at the distal end without fail through the transmission shaft, making it easier to fluidize the hard feces. Since the transmission shaft is housed within the flexible tube, the flexible tube and the transmission shaft can be formed as an integrated assembly, and can be interchangeably connected to the vibration generating unit. Therefore, every time disimpaction procedure is completed, the flexible tube and the transmission shaft may be disposed of and replaced with new ones.

According to an embodiment of the present invention, the insertion part and the liquid supply unit constitute a composite unit integrally formed as a single component. The composite unit includes a flexible tube interchangeably connected to the vibration generating unit, a vibration plate provided at the distal end of the flexible tube, and a liquid discharge port provided at the distal end of the flexible tube. With this configuration, the vibration generated by the vibration generating unit is transmitted without fail to the vibration plate at the distal end either through the flexible tube or through the liquid present inside the flexible tube, and liquid can be supplied from the discharge port at the distal end to the hard feces being vibrated.

The liquid supply source preferably includes a liquid container that holds the liquid and can be suspended, and a connecting flow passage through which the liquid flows down from the container into the flexible tube. By adjusting the suspension height of the liquid container, the fluid pressure of the liquid supplied from the liquid supply unit into the patient's rectum or anus can be regulated easily. Accordingly, the hard feces can be liquefied at a pressure appropriate for the patient. If the liquid supply unit is configured with a reservoir that is capable of storing the liquid and being pressurized to discharge the liquid and an enema container that includes a flexible tube tightly connected to the reservoir, the disimpaction device can be used in combination with an enema, which expands its range of applications.

The disimpaction set of the present invention integrates the above-mentioned disimpaction device and the storage bag for receiving feces. The storage bag includes a bag body and an adhesive part for attachment to the patient's buttocks. By attaching the storage bag around the patient's buttocks and using the disimpaction device, slurry-like feces discharged from the patient's anus can be collected in the storage bag. Accordingly, handling of slurry-like feces discharged from the anus of the patient becomes extremely clean and simple. The amount of removed feces can also be measured. Preferably, the storage bag includes an elastic part for opening that biases the opening of the bag body in an expanding direction. The elastic part for opening may be disposed along the upper edge portion of the bag body over a length exceeding half the circumference of the opening. With such a configuration, the opening of the bag body can be maintained in an open state by the elastic part for opening, thereby facilitating the discharge of slurry-like feces. When the adhesive part for attachment to buttocks is provided at a position overlapped with the elastic part for opening but over a range narrower than the elastic part for opening, the storage bag can be attached to the patient's buttocks while the opening of the bag body is held in an open state. When an easily bendable part is provided at an intermediate position in the circumferential direction of the elastic part for opening and the elastic part for opening on both sides of the easily bendable part is made contactable with each other, the storage bag can be stably folded into a flat shape by bending the elastic part for opening at the easily bendable part. When adhesive part for attachment to buttocks is provided on both sides of the easily bendable part, the storage bag can be attached to the patient's buttocks while the opening of the bag body is maintained in an open state.

### Advantageous Effects of Invention

According to the disimpaction device of the present invention, the number of components used is reduced, and hard feces can be vibrated while liquid is supplied to them, making it easier to further liquefy the hard feces. Moreover, because the number of components is small, the flexible tube can be replaced easily with a new one each time the disimpaction device is used. This improves the usability of the disimpaction device. In addition, by integrating a storage bag for feces, a disimpaction set that enables the feces-removal procedure to be performed easily can be provided.

### Brief Description of the Drawing

FIG. 1 is a side view of a disimpaction device according to a first embodiment of the present invention.
FIG. 2 describes the usage state of the disimpaction device according to the first embodiment.
FIG. 3 illustrates a vibration plate disposed at the distal end of an insertion part of the disimpaction device; (a) is a front view, and (b) is a side view.
FIG. 4 (a) is a cross-sectional view illustrating the state of use of the disimpaction device manufactured in the first embodiment, and FIG. 4 (b) is a cross-sectional view illustrating the state of use of an enema as performed in medical or nursing settings.
FIG. 5 is a side view illustrating a disimpation device according to a modification of the first embodiment.
FIG. 6 is a side view illustrating a disimpaction device according to a second embodiment of the present invention.
FIG. 7 illustrates a vibration plate disposed at the distal end of the insertion part of the disimpaction device shown in FIG. 7; (a) is a front view, and (b) is a side view.
FIG. 8 is a front view of a storage bag of a disimpaction set according to a third embodiment of the present invention.
FIG. 9 is a cross-sectional view of the part around the opening of the storage bag shown in FIG. 8.
FIG. 10 is a cross-sectional view illustrating the state where the storage bag in FIG. 8 is attached to buttocks.
FIG. 11 illustrates the state of use of the disimpaction set according to the third embodiment.

### Description of Embodiments

Several embodiments of the present invention will hereinafter be described in detail with reference to the drawings.

### [First Embodiment]

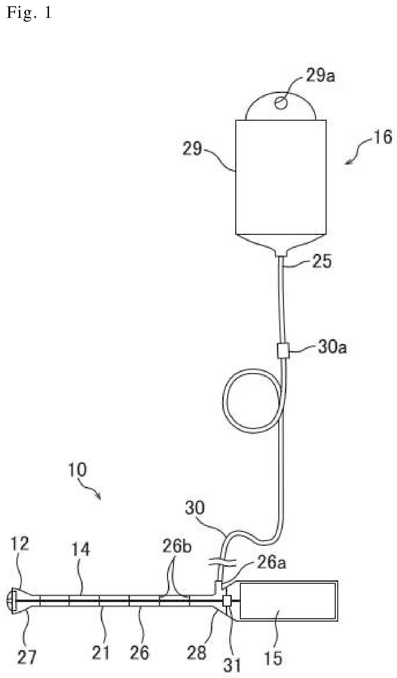

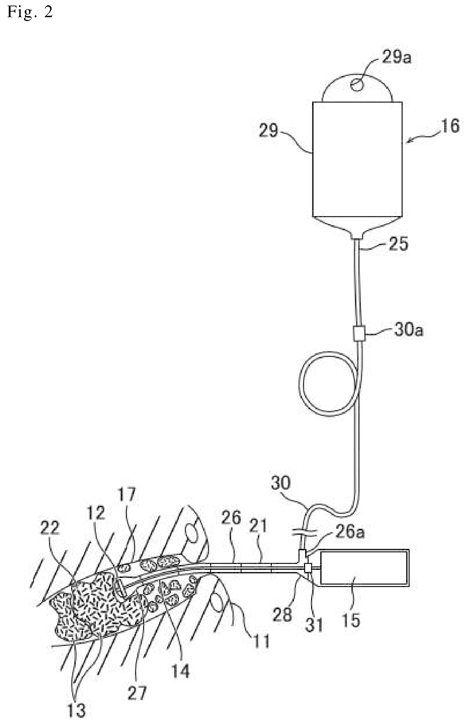

FIG. 1 is a side view illustrating a disimpaction device of the first embodiment, and FIG. 2 is a view illustrating its state of use. The disimpaction device 10 includes an insertion part 14 that is inserted through the anus 11 and whose distal end 12 is brought into contact with hard feces 13, a vibration generating unit 15 that is disposed outside the anus 11 and vibrates the distal end 12 of the insertion part 14, and a liquid supply unit 16 that supplies liquid from the distal end 12 to the hard feces 13.

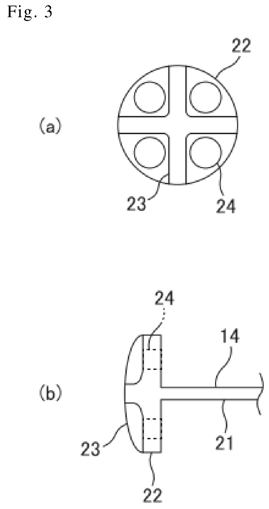

The insertion part 14 of this embodiment includes a flexible transmission shaft 21 that is connected to the vibration generating unit 15 and is capable of vibrating, and a vibration plate 22 attached to the distal end of the transmission shaft 21. The transmission shaft 21 is made of a rod-like material having flexibility. Vibrations from the vibrator 31 propagate along the entire length of the transmission shaft 21, thereby vibrating the vibration plate 22. During use, the transmission shaft 21 is inserted through the anus 11 into the rectum, and is allowed to bend and deform appropriately within the rectum, which may meander due to curvature or flexure. The material for the transmission shaft 21 is selected to have sufficient strength to withstand the applied vibration intensity and to avoid causing harm when inserted into the rectum. One end of the transmission shaft 21 is connected to the vibrator 31 of the vibration generating unit 15 in a detachable and easily replaceable manner. At the other end of the transmission shaft 21, a vibration plate 22 as shown in FIGS. 3 (a) and 3 (b) is connected. The shape of the vibration plate 22 is not particularly limited. In this embodiment, however, the vibration plate 22 is circular and has a protruding piece 23 that protrudes substantially in a cross shape along the axial direction of the transmission shaft 21. The protruding piece 23 is positioned at the front end of the vibration plate 22 so that it can directly contact the hard feces 13 during use. To the vibration plate 22, a liquid discharge port 24 passing through in the axial direction of the transmission shaft 21 is formed. This liquid discharge port 24 is a through-hole for supplying liquid from the liquid supply unit 16 to the hard feces 13. The transmission shaft 21 is housed within a flexible tube 26 of the liquid supply unit 16. The flexible tube 26 of this embodiment has, as described later, an expanded part 27 at its distal end, and the distal end of the expanded part 27 is open. The vibration plate 22 is accommodated in and disposed at the distal end of the expanded part 27. In this state, the protruding piece 23 of the vibration plate 22 curves outwards and protrudes externally.

The vibration generating unit 15 includes a vibrator 31 that oscillates by means of a drive unit such as a motor (not shown). The vibrator 31 may be configured so that it can be removed from the vibration generating unit 15 together with the transmission shaft 21 by disengaging the end of the transmission shaft 21 from the vibration generating unit 15. For example, a fitting hole may be provided in the vibrator 31 so that the end of the transmission shaft 21 can be fitted and secured therein. The method of joining the vibration generating unit 15 and the vibrator 31 is not particularly limited. The vibration generating unit 15 may have a structure similar to that of a general vibration device used by hand, such as an electric toothbrush, and may be a known device that can be readily realized without requiring any special techniques.

The vibration generated by the vibrator 31 of the vibration generating unit 15 may be a linear vibration along the transmission shaft 21, a rotational vibration around the transmission shaft 21, a vibration in a direction perpendicular to the transmission shaft 21, or a combination thereof. The vibration transmitted to the distal end 12 of the insertion part 14 must not cause any damage when it comes into contact with the anus 11 or the rectum 17. At the same time, the vibration transmitted to the distal end 12 of the insertion part 14 must be capable of breaking up the hard feces 13. The amplitude and frequency can be controlled or selected appropriately according to the patient's condition. As a general guideline, the vibration frequency may be approximately 1,000 to 10,000 cycles per minute, although this range is not strictly defined. Excessively high frequencies, such as ultrasonic frequencies, are not preferable because they make it difficult to break up the hard feces 13. Meanwhile, excessively low frequencies do not produce sufficient effect. The amplitude may generally be in the range of about 1 mm to 5 mm, although this value is not strictly defined. If the amplitude is too small, it becomes difficult to break up the hard feces 13, whereas if the amplitude is too large, it may cause damage to the inner wall of the anus 11 or rectum.

The liquid is supplied to the hard feces 13 from the distal end 12 of the insertion part 14 by the liquid supply unit 16. It is only necessary that the liquid is a flowable substance that causes no adverse effects to the patient and is capable of being mixed with the hard feces 13. The liquid may be water, for example, and physiological saline is ideal. The liquid supply unit 16 includes a liquid supply source 25 disposed outside the anus 11 and a flexible tube 26 that guides the liquid from the liquid supply source 25 to the distal end 12 of the insertion part 14. The flexible tube 26 accommodates the entire length of the transmission shaft 21 of the insertion part 14. The flexible tube 26 is formed of a resin that is capable of bending and deforming as appropriate along the curving or meandering shape of the rectum when inserted into the rectum together with the transmission shaft 21 through the anus 11.

The flexible tube 26 has, at one end, an end-connecting part 28 that is attachable to and detachable from the vibration generating unit 15, and at the other end, an expanded part 27 that surrounds and accommodates the peripheral region of the vibration plate 22 of the insertion part 14. The end-connecting part 28 and the expanded part 27 are liquid-tightly integrated into the intermediate portion of the tube. A supply port 26a for supplying liquid is provided on the side of the end-connecting part 28. It is preferable that the end-connecting part 28 be formed so that it can cover at least the outer periphery of the end of the vibration generating unit 15. The connection between the end-connecting part 28 and the vibration generating unit 15 must not allow leakage of liquid supplied into the flexible tube 26. Inside the intermediate portion of the flexible tube 26, a plurality of support portions 26b may be provided to support the transmission shaft 21 within a range that does not unduly impede its vibration. For example, the transmission shaft 21 may be supported around its outer periphery by the support portions 26b and then inserted together with the shaft 21 into the flexible tube 26 for installation. The flexible tube 26 configured as described above is replaced after each treatment.

The liquid supply source 25 includes a liquid container 29 capable of storing liquid and being suspended, and a connecting flow passage 30 that connects the liquid container 29 to the flexible tube 26. The liquid container 29 may be a resin bag having a part for suspension 29a, and liquid flows downward when the connecting flow passage 30 is connected to a spout at the lower end of the bag. The shape and size of the liquid container 29 may be selected as appropriate; in this embodiment, the container is a bag capable of holding approximately 500 cc of liquid. The connecting flow passage 30 can be made of various types of flexible tubing, having at one end a connecting part for the spout and at the other end a connecting part for liquid-tight attachment to the supply port 26a of the flexible tube 26. At the intermediate portion of the connecting flow passage 30, a flow-rate adjustment piece 30a is provided to regulate the flow rate of the liquid flowing through the connecting flow passage 30. With the liquid supply source 25, the pressure of the liquid being supplied is controlled by arbitrarily adjusting the suspension height of the part for suspension 29a.

To use the disimpaction device 10 of the present embodiment as described above, the patient is first placed in a lateral recumbent position, and the operator positions himself/herself behind the patient. In this state, as shown in Fig. 2, the insertion part 14 of the disimpaction device 10 is inserted together with the flexible tube 26 through the anus 11. When the insertion part 14 is inserted through the anus 11, the distal end of the disimpaction device 10 comes into contact with the hard feces 13. Next, when the vibration generating unit 15 is activated to vibrate the vibrator 31, the vibration propagates through the transmission shaft 21, causing the vibration plate 22 mounted to the distal end 12 to oscillate. At the same time, or prior to starting the vibration, the liquid from the liquid supply unit 16 flows through the flexible tube 26 and is discharged from the discharge port 24 of the vibration plate 22 positioned at the distal end 12 of the insertion part 14, and supplied to the vicinity of the hard feces 13. By the vibration of the vibration plate 22 and the supply of the liquid, the hard feces 13 in contact with them are deformed and crushed. Because liquid is supplied, the feces mix with moisture and become, so to speak, slurry-like feces. When the disimpaction device 10, which has been pressed forward, is pulled back, the front part of the disimpaction device 10-namely, the area near the vibration platecomes under negative pressure. As the disimpaction device 10 is pulled back, liquid is replenished from the distal end. When the disimpaction device 10 is then advanced again, the hard feces 13 and the liquid readily mix, turning the feces into slurry. The liquefied, slurry-like feces flow easily out of the anus 11. In actual use, it is preferable to proceed while repeatedly making small forward and backward movements.

After completing the disimpaction procedure, the insertion part 14 of the disimpaction device 10 is withdrawn from the patient's body, and the flexible tube 26 of the liquid supply unit 16, the transmission shaft and the vibration plate 22 of the insertion part 14, and other components are removed from the vibration generating unit 15 and discarded as consumables. The liquid container 29 and the connecting flow passage 30 of the liquid supply unit are also discarded as consumables. A new insertion part 14 and a liquid supply unit 16 can then be attached to the vibration generating unit 15 for use in the next procedure.

The safety of the disimpaction device 10 according to the present embodiment will hereinafter be described.

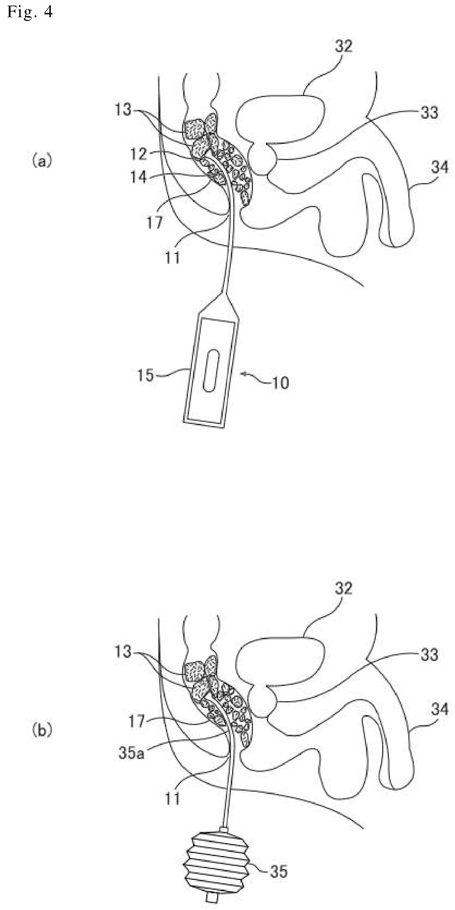

FIG. 4 (a) is a cross-sectional view showing the state of use of the disimpaction device 10 manufactured in this embodiment, and FIG. 4 (b) is a cross-sectional view showing the state of use of an enema as performed in medical or nursing settings. In the figures, 32 denotes the bladder, 33 the prostate, and 34 the penis. In FIG. 4 (a), the flexible tube 26 of the disimpaction device 10 is inserted through the anus 11, and the distal end of the flexible tube 26 is positioned within the feces inside the rectum 17. Meanwhile, FIG. 4 (b) shows the flexible tube 35a of an enema 35 inserted through the anus 11, with the distal end of the flexible tube 35a positioned within the feces inside the rectum 17.

As can be seen by comparing FIG. 4 (a) and FIG. 4 (b), the state of use of the disimpaction device 10 of the present embodiment and that of the enema 35 are almost identical in configuration. The flexible tube 26 inserted through the anus 11 in the disimpaction device 10 and the flexible tube 35a inserted through the anus 11 in the enema 35 are made of the same type of flexible material and are nearly identical in flexibility, size, and shape. Therefore, the disimpaction device 10 of the present embodiment is sufficiently compatible with the human body and can be used safely. Some may feel that large amounts of discharged slurry-like feces are unsanitary or troublesome to handle. However, it should be emphasized that this is not the case. In conventional feces extraction or enema procedures, which rely on manual techniques, feces inevitably adhere to the operator's fingers, requiring the use of considerable amounts of paper. In contrast, the disimpaction device 10 of the present embodiment liquefies the hard feces 13 into slurry from the outset and discharges it through the anus 11, thereby eliminating the need for manual intervention.

According to the disimpaction device 10 of the present embodiment, the distal end 12 of the insertion part 14 is inserted through the anus 11 and brought into contact with the hard feces 13, and liquid is supplied from the liquid supply unit 16. By vibrating the distal end of the insertion part 14 with the vibration generating unit 15, the hard feces 13 and the liquid are mixed. This enables the hard feces 13 present in the rectum and anus 11 to be liquefied, preferably into slurry, forming slurry-like feces. Because the insertion part 14 inserted from the anus 11 into the rectum 17 has flexibility that allows it to deform along the shape of the rectum 17, it can be inserted easily and safely into the patient's rectum, facilitating the feces extraction procedure. Reducing the workload of difficult feces extraction procedure offers significant social benefits. Even if the feces are dissolved slowly, the working time can ultimately be shortened if the procedure proceeds reliably. When the disimpaction device 10 operates smoothly, the procedure can be performed reliably, safely, and hygienically, providing considerable peace of mind to both the operator and the patient. Furthermore, the shape, size, and softness of the disimpaction device can be made approximately equivalent to that of a glycerin enema. The vibration at the distal end 12 of the insertion part 14 is not excessive and does not cause harm even if it comes into contact with the mucosa of the large intestine, making it safe to be used for the human body.

The disimpaction device 10 of the present embodiment includes a flexible tube 26 that guides the liquid from the liquid supply source 25 to the distal end 12 of the insertion part 14, and the vibration plate 22 is provided with a protruding piece 23 that protrudes in the axial direction from the surface facing the hard feces and makes direct contact with the hard feces. The vibration plate 22 is positioned at the distal end of the flexible tube 26 and has a liquid discharge port 24 through which the liquid from the flexible tube 26 is discharged. Accordingly, the liquid can be reliably supplied to the distal end of the insertion part 14 from outside the anus, and by supplying the liquid from the distal end 12 and vibrating the vibration plate 22 while it is held in axial contact with the hard fecal mass, the mass is crushed, allowing the feces and the liquid to mix and form slurry-like feces.

The insertion part 14 includes a flexible, vibratable transmission shaft 21 connected to the vibrator 15, and a vibration plate 22 provided at the distal end 12 of the transmission shaft 21. Accordingly, the vibration generated by the vibrator located outside the anus 11 is reliably transmitted to the vibration plate 22 at the distal end through the transmission shaft 21, making it easier to liquefy the hard feces 13. The flexible tube 26 and the transmission shaft 21 may be formed integrally, and these components-the transmission shaft 21 and the flexible tube 26-are connected to the vibration generating unit 15 in a replaceable state. Therefore, each time the disimpaction device 10 is used, the flexible tube 26 and the transmission shaft 21 can be disposed of and easily replaced with new ones.

When the liquid supply source 25 includes a liquid container 29 capable of storing liquid and being suspended, and a connecting flow passage 30 through which the liquid can flow from the liquid container 29 to the flexible tube 26, the liquid pressure of the liquid supplied from the liquid supply unit 16 into the patient's rectum or anus 11 can be easily adjusted by regulating the suspension height of the liquid container 29. As a result, the hard feces 13 can be liquefied at a liquid pressure suitable for the patient.

### Modification of the First Embodiment

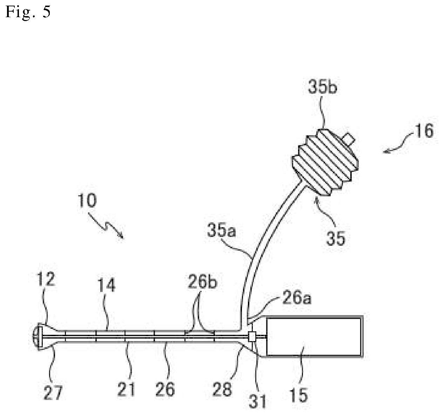

FIG. 5 is a side view of the disimpaction device 10 according to a modification of the first embodiment. According to the first embodiment of the disimpaction device 10, it is possible to remove fecal masses located up to about 10 cm from the anus. However, some patients may feel that there are still fecal masses deeper in the rectum 17 and may request an enema. Therefore, this modification is proposed. In other words, instead of the liquid container 29 containing water or physiological saline for liquefying the hard feces 13, an enema container with glycerin enema solution is integrated, making it easy to continuously perform the enema procedure.

In this modification, the liquid supply unit 16 has a reservoir 35b that can store the enema liquid and discharge it by pressurization, and a flexible tube 35a that is liquid-tightly connected to the reservoir 35b. The enema 35 is attached to the disimpaction device 10 in place of the liquid container 29 and the connecting flow passage 30 of the first embodiment. The reservoir 35b is made of a flexible resin, and the enema liquid stored inside can be pushed out into the flexible tube 35a by pressing the bellows-shaped outer wall with fingers. The flexible tube 35a is connected at one end to the reservoir 35b, and the other end is connected to the supply port 26a of the flexible tube 26, which is detachably coupled to the vibration generating unit 15. Except for these points, the configuration is the same as the first embodiment.

Even with this disimpaction device 10, the above-described operational effects of the first embodiment can be obtained. Moreover, since the liquid supply unit 16 has an enema 35 that includes a reservoir 35b, which stores the enema liquid and discharges it by being pressurized, and a flexible tube 35a that is liquid-tightly connected to the reservoir 35b, a known enema device can be combined with the disimpaction device 10, thereby expanding the range of applications.

### Second Embodiment

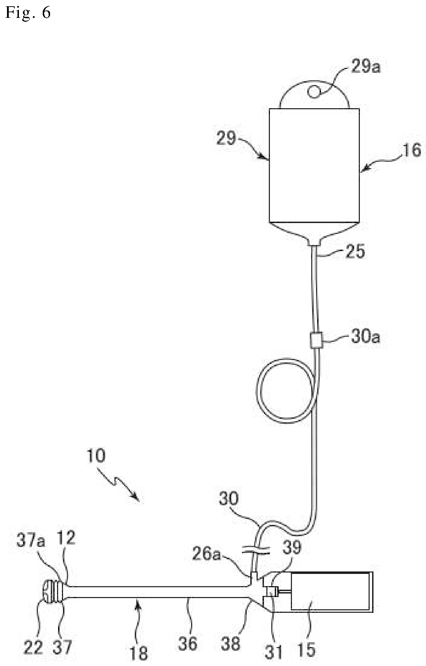

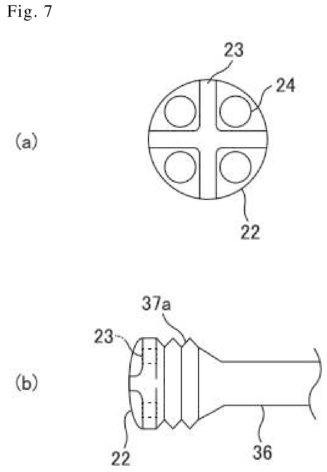

The second embodiment of the present invention will then be described. The inventor first devised the structure of the first embodiment, in which vibrations are transmitted using a transmission shaft. After a great deal of consideration, however, it was found that vibrations could be transmitted by a liquid within the flexible tube without using a transmission shaft, and thus the structure of this second embodiment was invented. Figure 6 is a side view of the disimpaction device according to the second embodiment, and FIGS. 7 (a) and 7 (b) show the distal end of a composite unit. In the disimpaction device 10 of the second embodiment, the insertion part and the liquid supply unit are integrally formed as a composite unit 18. This composite unit 18 includes a flexible tube 36 that is detachably connected to the vibration generating unit, a vibration plate 22 provided at the distal end 37 of the flexible tube 36, and a liquid discharge port 24 provided at the distal end 37 of the flexible tube 36.

In the composite unit 18, the flexible tube 36 has, on one end, a terminal coupling part 38 that is configured to be attachable to and detachable from the vibration generating unit 15 so as to be replaced. On the other end, a bellows part 37a is provided around the outer periphery, and a vibration plate 22 is provided at the distal end. The terminal coupling part 38 covers the outer periphery of the end of the vibration generating unit 15. When connecting the terminal coupling part 38 to the vibration generating unit 15, the connection is made liquid-tight to the extent that liquid supplied into the flexible tube 26 will not leak. A supply port 26a for supplying liquid is provided on the side of the terminal coupling part 38. The vibration plate 22 that seals the distal end of the flexible tube 36 has the same structure as in the first embodiment; it is substantially circular and has a protruding piece 23 that protrudes in a cross shape. The vibration plate 22 is provided with discharge ports 24 for supplying liquid to the hardened feces 13. In the drawings, four discharge ports 24 are shown, but the number, shape, size, and other features of the discharge ports 24 can be suitably modified.

The vibration generating unit 15 has, at the end connected to the flexible tube 36, a cylindrical hollow portion 39, in which a piston-shaped vibrator 31 is disposed in a liquid-tight manner. The hollow portion 39 accommodates the liquid supplied into the flexible tube 36, and the vibrator 31 is in contact with the liquid. In the second embodiment, when the liquid is supplied from the liquid container 29 into the flexible tube 36 through the supply port 26a, the liquid travels through the flexible tube 36 to the distal end 37 and is supplied from the discharge ports 24 of the vibration plate 22. At the same time, the liquid inside the flexible tube 36 fills the hollow portion 39 of the vibration generating unit 15 so that it is constantly in contact with the piston-shaped vibrator 31. Accordingly, when the vibrator 31 vibrates, the vibration propagates through the liquid inside the flexible tube 36, causing the distal end 37 of the flexible tube 36 to vibrate back and forth and also causing the bellows part 37a to vibrate. When vibration is transmitted through the liquid, it is preferable to modify the shape of the discharge ports 24-for example, a valve may be provided or a port in an orifice shape may be provided. The vibration of the vibrator 31 may also be propagated to the bellows part 37a and the vibration plate 22 at the distal end 37 of the flexible tube 36. In any of these modes, the vibration from the vibration generating unit 15 can be propagated to vibrate the bellows part 37a and the vibration plate 22. Other aspects are the same as in the first embodiment, and the device can be used in the same manner.

According to the disimpaction device 10 of the second embodiment, the same operational effects as in the first embodiment can be obtained. With this disimpaction device 10, the composite unit 18 and the liquid supply unit 16 are integrated into one, and the device includes a flexible tube 36 that is detachably connected to the vibration generating unit 15, a vibration plate 22 provided at the distal end 37 of the flexible tube 36, and liquid discharge ports 24 provided at the distal end 37 of the flexible tube 36. Accordingly, vibration from the vibration generating unit 15 can be transmitted to the distal end 37 through the flexible tube 36 and the liquid inside the flexible tube 36, allowing the vibration plate 22 at the distal end 37 to apply vibration to the hardened feces 13. In addition, liquid can be supplied to the distal end 37 through the flexible tube 36 and delivered to the hardened feces 13 to be vibrated through the discharge ports 24 of the vibration plate 22. Thus, hardened feces 13 can be liquefied with fewer components. In particular, because the insertion part of the flexible tube 36 and the liquid supply unit are integrated as the composite unit 18, the number of parts discarded after each use is reduced, allowing the device to be provided at a lower cost compared to the disimpaction device 10 of the first embodiment.

The above embodiments may be modified as appropriate within the scope of the present invention. In the embodiments described above, a typical vibration plate 22 was shown, but vibration plates of various shapes may be used. Although an example was given in which the supply port 26a of the liquid supply unit 16 is provided on the flexible tube 26, the invention is not limited thereto. For example, the supply port 26a of the liquid supply unit 16 may instead be provided on the vibration generating unit 15.

### Third Embodiment

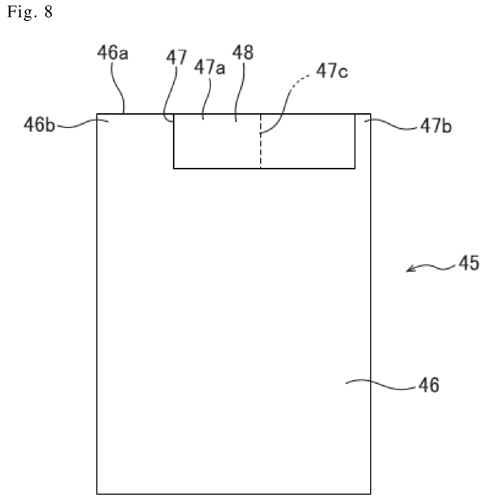

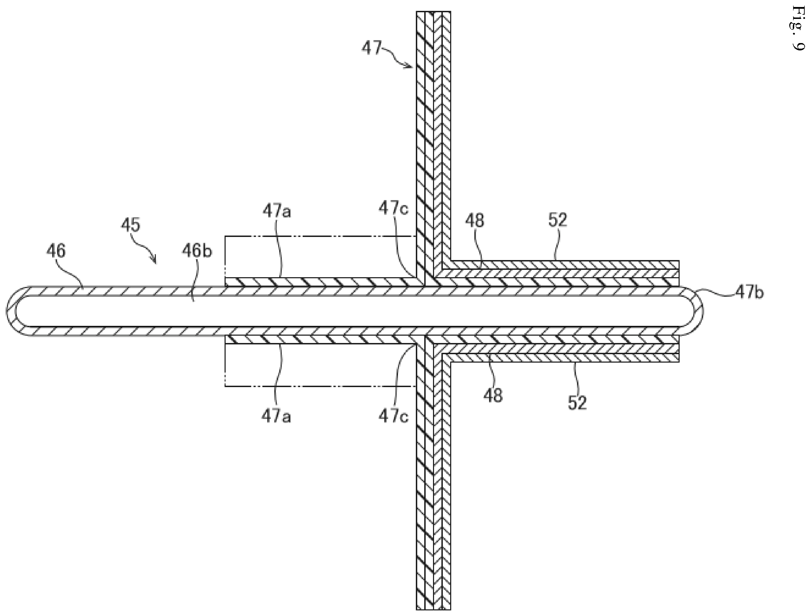

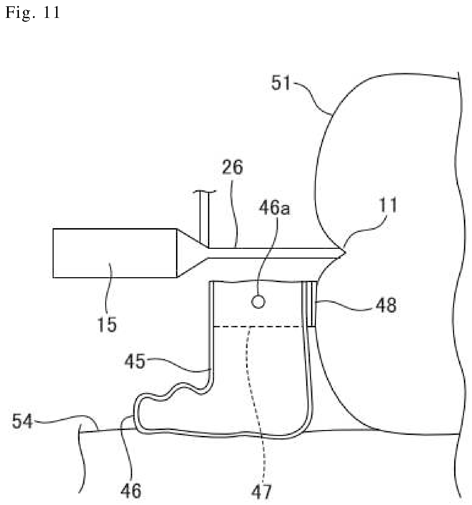

Next, a disimpaction set will be described with reference to FIGS. 8 to 11. FIG. 11 shows the disimpaction set in its state of use. The disimpaction set of this embodiment includes any one of the above-described disimpaction devices 10 and a storage bag 45 for storing the extracted feces. As shown in FIGS. 8 and 9, the storage bag 45 includes a bag body 46, an elastic part for opening 47, and an adhesive part for attachment to buttocks 48. The bag body 46 is made of a transparent flexible resin, and an opening 46b is provided at its upper edge portion 46a.

The elastic part for opening 47 is used to fit the bag body 46 onto the patient's buttocks 51 by biasing the opening 46b of the bag body 46 in an expanding direction, and is provided on the upper edge portion 46a along the opening 46b of the bag body 46. The elastic part for opening 47 is a thin elastic member having elasticity at least greater than that of the bag body 46, and may be made of a resin, such as polypropylene for example, having a level of elasticity that allows users to easily deform it with their fingers. The elastic part for opening 47 elastically deforms into a curved shape when pressed in the thickness direction against its elasticity and returns to a flat shape when the pressure is released. The elastic part for opening 47 is attached to the outer peripheral surface of the bag body 46 and is arranged over a range longer than half the circumference of the opening 46b of the bag body 46. In this embodiment, an easily bendable part 47b is provided as a slight gap at an intermediate position in the circumferential direction of the elastic part for opening 47. The elastic part for opening 47 of this embodiment is configured by arranging two elastic members 47a side by side in the circumferential direction with the easily bendable part 47b interposed in between. Each elastic member 47a is formed by partially joining two strip-shaped elastic pieces. Specifically, a folding part 47c is formed at an intermediate position of each elastic piece by bending, and the outer surface of each of the elastic piece is valley-folded at the folding part 47c to bring both sides together, facing each other. The elastic piece forming each elastic member 47a is shorter than half the circumference of the opening 46b of the bag body 46, and the elastic part for opening 47 as a whole is formed to have a length longer than half the circumference of the opening 46b of the bag body 46.

When not in use, the storage bags are folded into a thin, flat shape and stored in stacked form. It is only necessary that the easily bendable part 47b of the storage bag is bent in the closing direction so that the two elastic members 47a facing each other across the easily bendable part 47b overlap each other. That is, as shown in FIG. 9, the storage bag 45 is folded flat by mountain-folding the easily bendable part 47b so that the inner walls of the bag body 46 face each other. In this state, each elastic member 47a can be valley-folded at the folding part 47c provided at an intermediate position in the longitudinal direction, as indicated by the imaginary lines in FIG. 9. As a result, the entire storage bag 45 is maintained in a flat, planar state.

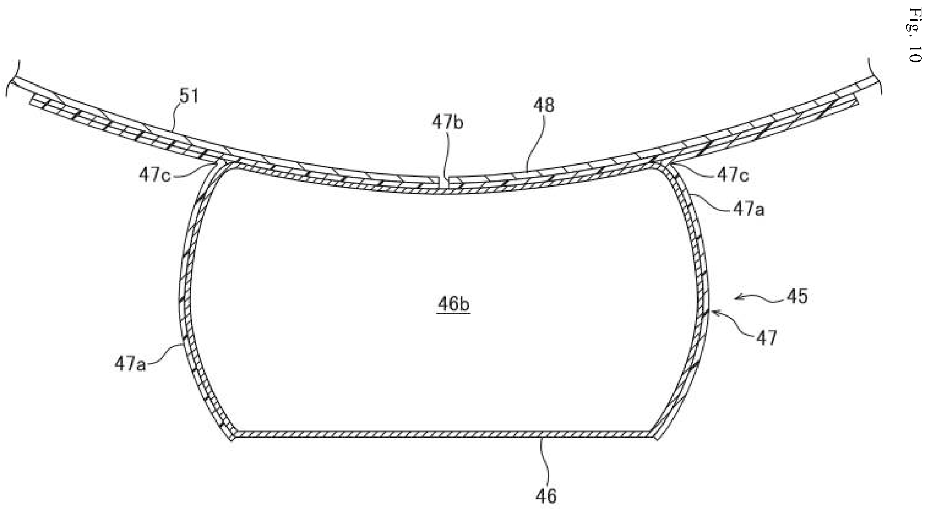

On the other hand, during use, the elastic pieces of each elastic member 47a are unfolded at the location of the folding part 47c, and the easily bendable part 47b is further opened. As a result, the elastic part for opening 47, which is arranged on both sides of the easily bendable part 47b and joined to the bag body 46, expands. As shown in FIG. 10, by pulling the elastic part 47 on both sides to the left and right, the opening 46b of the bag body 46 opens accordingly, allowing the elastic pieces of each elastic member 47a to be deformed so as to conform to the patient's buttocks 51.

The adhesive part for attachment to buttocks 48 is used to affix the bag body 46 to the patient's buttocks 51 and is provided along the outer peripheral surface of the upper edge portion 46a that includes the opening 46b of the bag body 46. The adhesive part for attachment to buttocks 48 is a strip-shaped doublesided adhesive tape for example, the surface of which is covered along its entire length with release paper 52. During use, the adhesive part for attachment to buttocks 48 is attached to the patient's buttocks 51 at a position where it is secured. Specifically, the adhesive part for attachment to buttocks 48 is attached to the elastic pieces of the two elastic members 47a arranged on both sides of the easily bendable part 47b and is secured to the buttocks 51 when the elastic pieces of the elastic members 47a are unfolded at the folding part 47c. It is only necessary that the adhesive strength of the adhesive part for attachment to buttocks 48 is sufficient to secure the side of the opening 46b of the bag body 46 to the patient's buttocks 51 while the bag body 46 contains sludge-like feces during use.

To use the disimpaction set, which includes the storage bag 45 and any one of the above-described disimpaction devices, the storage bag 45 is attached by adhering the adhesive part for attachment to buttocks 48 to the patient's buttocks 51 while the opening 46b of the storage bag 45 is in an expanded state, as shown in FIGS. 10 and 11. When not in use, the storage bag 45 is stored in a flat, folded state together with the elastic part for opening 47, as shown in FIGS. 8 and 9, at the location of the easily bendable part 47b of the elastic part for opening 47, and each elastic member 47a is also folded at its respective folding part 47c for storage. The storage bag 45 is unfolded from this folded state, and the opening 46b is expanded together with the elastic part for opening 47.

As shown in FIGS. 10 and 11, the patient is placed in a lateral recumbent position on the treatment table 54. After the release paper 52 is peeled from the storage bag 45, the adhesive part for attachment to buttocks 48 is applied to the patient's buttocks 51 at a position directly below the anus. This causes the opening 46b of the bag body 46 to naturally open and be positioned facing upward directly beneath the anus. Specifically, as shown in FIG. 10, the two elastic members 47a, which are arranged on both sides of the easily bendable part 47b of the elastic part for opening 47, are unfolded from the posterior side toward the anterior side of the patient's buttocks 51 and adhered to the buttocks 51 by the adhesive part for attachment to buttocks 48. Here, because the two elastic pieces of the elastic part for opening 47 arranged along the outer periphery of the opening 46b of the bag body 46 are each set to have a total length longer than half the circumference of the opening 46b, the elastic pieces of the two elastic members 47a bias the opening 46b in a direction that expands it. As a result, the opening 46b is naturally maintained in an expanded state, as shown in FIG. 10.

After attaching the storage bag 45 to the patient's buttocks 51 in this manner, the hard feces 13 are removed using the disimpaction device 10, as shown in FIG. 11. When the insertion part 14 of the disimpaction device 10 is inserted, together with the flexible tube 26, through the anus 11 and guided to the fecal mass, the vibration generating unit 15 is activated to vibrate the vibration plate 22 at the distal end 12 of the insertion part 14. At the same time, or prior to the start of vibration, liquid is discharged from the distal end 12 of the insertion part 14 through the flexible tube 26 from the liquid supply unit 16 and supplied to the area around the hard feces 13.

As a result, the hard feces 13 are gradually broken down and mixed with moisture, forming sludge-like feces. The liquefied sludge-like feces then flow out through the anus 11. At this time, the sludge-like feces discharged from the anus flow downward into the storage bag 45 attached to the patient's buttocks 51 and is collected there. Because the opening 46b of the storage bag 45 is kept open by the elastic part for opening 47 and positioned directly below the anus, the sludge-like feces can be collected cleanly and without leakage during the procedure.

After the procedure is completed, the insertion part 14 of the disimpaction device 10 is withdrawn from the patient's body, and components such as the flexible tube 26 of the liquid supply unit 16 and the transmission shaft as well as the vibration plate 22 of the insertion part 14 are removed from the vibration generating unit 15 and discarded as consumables. Meanwhile, the storage bag 45 that has collected the sludge-like feces can be removed from the patient's buttocks 51 by peeling off the adhesive part for attachment to buttocks 48, and then discarded. By measuring the weight of the storage bag 45 containing the sludge-like feces and subtracting the weight of the liquid supplied from the liquid supply unit 16, the weight of the extracted feces can be accurately calculated. In this process, if an opening 47b is provided near the upper edge portion 46a of the bag body 46, the bag can be suspended for weight measurement.

According to the above disimpaction set, when using the disimpaction device 10, the storage bag 45 is adhered around the patient's buttocks 51, allowing the sludge-like feces discharged from the patient's anus 11 to be collected in the storage bag 45 without requiring any separate cleaning procedure. Therefore, handling the sludge-like feces discharged from the anus 11 is extremely sanitary and simple, and the amount of extracted feces can also be measured. In addition, there is no need to use large amounts of paper. By collecting the sludge-like feces that flow out of the patient's buttocks in the storage bag, handling of feces becomes very clean and convenient.

In this third embodiment, the elastic part for opening 47, which is provided along the upper edge portion 46a of the bag body 46 over a range longer than half the circumference of the opening 46b, biases the opening 46b in the expanding direction. Therefore, the opening 46b of the bag body 46 is maintained in an open state, and the bottom of the bag body rests stably on the treatment table, allowing the disimpaction procedure to be performed without the need for an assistant.

Because the adhesive part for attachment to buttocks 48 is provided in a range narrower than the elastic part for opening 47, and at a position overlapping the elastic part for opening 47, the storage bag 45 can be adhered to and attached onto the patient's buttocks 51 while keeping the opening 46b of the bag body 46 widely expanded. When not in use, the elastic part for opening 47 on both sides of the easily bendable part 47b can be overlapped and folded, enabling the storage bag 45 to be kept in a flat state.

The third embodiment may be modified as appropriate within the scope of the present invention. For example, the elastic members 47a may be arranged as one continuous integral piece without being divided into two. In that case, it is sufficient to form a folded part bent 180 degrees at an intermediate position as the easily bendable part 47b, provided that the folded part can be unfolded by 180 degrees. The elastic part for opening 47 may also be provided on the inner peripheral surface of the upper edge portion 46a of the bag body instead of on the outer peripheral surface.

### Reference Signs List

- 10: Disimpaction device
- 11: Anus
- 12: Distal end
- 13: Hard feces
- 14: Insertion part
- 15: Vibration generating unit
- 16: Liquid supply unit
- 17: Rectum
- 18: Composite unit
- 21: Transmission shaft
- 22: Vibration plate
- 23: Protruding piece
- 24: Discharge port
- 25: Liquid supply source
- 26, 36: Flexible tube
- 26a: Supply port
- 26b: Support portion
- 27: Expanded part
- 28: End-connecting part
- 29: Liquid container
- 29a: Part for suspension
- 30: Connecting flow passage
- 30a: Flow rate adjustment piece
- 31: Vibrator
- 32: Bladder
- 33: Prostate
- 34: Penis
- 35: Enema
- 35a: Flexible tube
- 35b: Reservoir
- 37: Distal end
- 37a: Bellows part
- 38: Terminal coupling part
- 39: Hollow portion
- 42: Introductory portion
- 45: Storage bag
- 46: Bag body
- 46a: Upper edge portion
- 46b: Opening
- 47: Elastic part for opening
- 47a: Elastic member
- 47b: Easily bendable part
- 47c: Folding part
- 48: Adhesive part for attachment to buttocks
- 51: Buttocks
- 52, 53: Release paper
- 54: Treatment table

## Claims

1. A disimpaction device, comprising:
an insertion part configured to be inserted through an anus and bring a distal end thereof into contact with hard feces;
a vibration generating unit disposed outside the anus and configured to vibrate the distal end of the insertion part; and
a liquid supply unit configured to supply liquid to the hard feces from the distal end of the insertion part,
wherein, the insertion part is flexible so as to deform along a shape of a rectum when inserted from the anus into the rectum; and
vibration of the distal end of the insertion part mixes the hard feces with the liquid to form sludge-like feces.

2. The disimpaction device according to claim 1, wherein the liquid supply unit comprises a liquid supply source disposed outside the anus and a flexible tube that guides liquid from the liquid supply source to the distal end of the insertion part;
a vibration plate is disposed at the distal end of the insertion part, and the vibration plate is provided with a protruding piece that protrudes in the axial direction from the surface facing the hard feces so as to come into direct contact with the hard feces; and
the vibration of the vibration plate mixes the hard feces with the liquid to form the slurry-like feces.

3. The disimpaction device according to claim 1, wherein the vibration generating unit generates vibrations having an amplitude of 1 mm to 5 mm.

4. The disimpaction device according to claim 2, wherein the vibration plate is disposed at the distal end of the flexible tube, and the vibration plate includes a liquid discharge port through which the liquid guided from the flexible tube is discharged.

5. The disimpaction device according to claim 4, wherein the insertion part comprises a flexible transmission shaft that is connected to the vibration generating unit and is capable of vibrating, and the vibration plate connected to the transmission shaft; the transmission shaft is housed within the flexible tube; and the transmission shaft and the flexible tube are detachably connected to the vibration generating unit.

6. The disimpaction device according to claim 4, wherein the insertion part and the liquid supply unit are integrally formed as a composite unit; and
the composite unit comprises the flexible tube detachably connected to the vibration generating unit and the vibration plate disposed at the distal end of the flexible tube.

7. The disimpaction device according to claim 1, wherein the liquid supply source comprises a liquid container that holds the liquid and can be suspended, and a connecting flow passage through which the liquid can flow from the liquid container to the flexible tube.

8. The disimpaction device according to claim 5, wherein the liquid supply unit is an enema comprising a reservoir that stores the liquid and can discharge the liquid by being pressurized and the flexible tube liquid-tightly connected to the reservoir.

9. A disimpaction set, comprising: a disimpaction device according to any one of claims 1 to 8; and a storage bag for storing feces, wherein the storage bag includes a bag body and an adhesive part for attachment to buttocks that allows the bag body to be affixed to buttocks.

10. The disimpaction set according to claim 9, wherein the adhesive part for attachment to buttocks is provided along the upper edge portion of the bag body.

11. The disimpaction set according to claim 10, wherein the storage bag is provided with an elastic part for opening that biases the opening of the bag body in an expanding direction.

12. The disimpaction set according to claim 11, wherein the elastic part for opening is disposed along an upper edge portion of the bag body over a range longer than half the circumference of the opening of the bag body.

13. The disimpaction set according to claim 12, wherein the adhesive part for attachment to buttocks is provided so as to overlap the elastic part for opening over a range narrower than the elastic part for opening.

14. The disimpaction set according to claim 13, wherein the elastic part for opening comprises an easily bendable part at an intermediate position in the circumferential direction; and the elastic part for opening located on both sides of the easily bendable part can be made to face and abut against each other.

15. The disimpaction set according to claim 14, wherein the adhesive part for attachment to buttocks is provided on both sides of the easily bendable part.
